# EUROPÄISCHE PATENTSCHRIFT

(11) **EP 0 387 527 B1**
(45) Veröffentlichungstag und Bekanntmachung des Hinweises auf die Patenterteilung: **22.06.1994**
(21) Anmeldenummer: 90102685.6
(22) Anmeldetag: 12.02.1990
(51) Int. Cl.: C12N 15/11, C12P 13/08, C12N 1/21, C07H 21/04

(54) **Verfahren zur fermentativen Herstellung von L-Lysin**
Process for the fermentative production of L-lysine
Procédé pour la production fermentative de L-lysine

(30) Priorität: 14.03.1989 DE 3908201
(43) Veröffentlichungstag der Anmeldung: 19.09.1990
(73) Patentinhaber: Degussa Aktiengesellschaft, 60311 Frankfurt (DE)
(72) Erfinder: Bachmann, Bernd, Dr., D-4806 Werther (DE); Thierbach, Georg, Dr., D-4800 Bielefeld (DE); Kalinowski, Jörn, D-4800 Bielefeld (DE); Pühler, Alfred, Prof. Dr., D-4800 Bielefeld (DE)

(56) Entgegenhaltungen:
- EP-A- 88 166
- EP-A- 197 335
- EP-A- 219 027

## Beschreibung

Die Erfindung betrifft ein Verfahren zur fermentativen Herstellung von L-Lysin.

Corynebacterium glutamicum und verwandte Gattungen wie z. B. Brevibacterium lactofermentum und Brevibacterium flavum sind als Aminosäuren bildende Mikroorganismen bekannt.

Um die Produktivität zu erhöhen, führt man künstliche Mutationen durch.

Beispiele für so erzeugte künstliche Mutanten sind z. B. Lysin produzierende Stämme von Corynebacterium glutamicum, die neben einer AEC-Resistenz (AEC ≙ S-2-Aminoethylcystein) eine damit gekoppelte Homoserin- und Leucin-Auxotrophie (US-PS 3 708 395) zeigen oder sensitiv gegenüber Methionin sind (US-PS 3 871 960).

Neben dieser klassischen Methode wurden Vektorsysteme entwickelt, die die Transformation von Mikroorganismen der Gattungen Corynebacterium und Brevibacterium ermöglichen (DE-OS 3737719, DE-OS 3841453, Thierbach G., Schwarzer A., Pühler A, Appl. Microbiol. Biotechnol. 29 (1988) 356-362).

In der EP-A-0219 027 wird ein Verfahren zur Herstellung verschiedener Aminosäuren beschrieben, bei dem man mit rekombinanter DNA Mikroorganismen der Gattungen Corynebacterium und Brevibacterium transformiert und so die Ausscheidungsmenge von Aminosäuren erhöht.

Die rekombinante DNA enthält dabei ein für die Synthese von Aspartatsemialdehyd-Dehydrogenase oder Aspartataminotransferase kodierendes DNA-Fragment.

Aus der US-PS 4,346,170 ist die Klonierung einer die Lysinbildung kontrollierenden genetischen Information in E. coli bekannt, die aus einem Stamm derselben Gattung mit einer Resistenz gegen eine L-Lysin-analoge Verbindung wie z.B. AEC stammt.

Der Gegenstand der US-PS 4,560,654 liegt auf demselben Gebiet. In diesem Fall wird jedoch in einem Lysin-auxotrophen Stamm von Corynebacterium glutamicum eine genetische Information aus einem AEC-resistenten Stamm derselben Gattung kloniert mit der Folge, daß Lysin ausgeschieden wird.

Die Identität des kloniertenDNA-Fragments wird nicht offenbart.

Auch der EP-A-88166 ist nur zu entnehmen, daß ein Stamm von C.glutamicum Lysin ausscheidet, nachdem er durch Tranformation den Phänotyp der AEG-Resistenz erworben hat.

Das für diesen Zweck eingesetzte rekombinante Plasmid pAec5 enthält ein 3,9 kb Fragment chromosomaler DNA eingefügt an der BglII-Schnittstelle des Vektors pCG 11.

Aufgabe der Erfindung ist, die Regulierbarkeit eines wichtigen Enzyms der Lysin-Biosynthese in einem Mikroorganismus der Gattung Corynebacterium oder Brevibacterium so zu verändern, daß entweder eine Lysin-Ausscheider resultiert oder die Rate der Lysin-Ausscheidung erhöht wird.

Die Erfindung betrifft ein Verfahren zur Herstellung von L-Lysin, das dadurch gekennzeichnet ist, daß man rekombinante DNA, die aus einem DNA-Fragment, das eine für die Produktion von Proteinen, die zu einer Aspartyl-β-semialdehyd-Dehydrogenase (asd) Aktivität und zur Deregulation der Aspartat-Kinase (lysC) führen, kodierende genetische Sequenz aufweist, die von einem Mikroorganismus der Gattung Corynebacterium oder Brevibacterium stammt, und aus Vektor DNA besteht, in einen gegebenenfalls Lysin-produzierenden Mikroorganismus der Gattung Corynebacterium oder Brevibacterium inseriert, den so erhaltenen Transformanten in einem geeigneten, an sich bekannten Medium züchtet und das gebildete L-Lysin daraus mit bekannten Methoden abtrennt.

Als Donorstämme können alle, bevorzugt L-Lysin produzierende Bakterien der Gattung Brevibacterium und Corynebacterium dienen, die die entsprechenden DNA-Sequenzen enthalten, insbesondere aber Corynebacterium glutamicum DM 58-1, das durch Mutagenese von Corynebacterium ATCC 13032 mit Ethylmethansulfonat entwickelt wurde und AEG-Resistenz zeigt.
Dieser Stamm ist unter der Nummer DSM 4697 hinterlegt, wo er als Wirtsbakterium für das Plasmid pDM6 dient. Dieses kann der Fachmann nach bekannten Verfahren abtrennen und so den Stamm DM58-1 erhalten. (FEMS Microbiology Review 32 (1986) 149-157)
Die chromosomale DNA wird aus dem Donor auf bekannte Weise extrahiert und mit Restriktionsendonucleasen behandelt.
Nach der Konstruktion der rekombinanten DNA durch Einführung des chromosomalen DNA-Fragments in einen Vektor erfolgt die Transformation des Mikroorganismus mit dem so gewonnenen Plasmid, erfindungsgemäß beispielsweise mit pCS2, dessen Restriktionskarte in Abb. 2 dargestellt ist, und das in dem Stamm Corynebacterium glutamicum DM2-1/pCS2 unter der Nummer DSM 5086 bei der Deutschen Sammlung für Mikroorganismen und Zellkulturen nach dem Budapester Abkommen hinterlegt wurde.

Ein bevorzugtes Vektorsystem stellt pZ1 (hinterlegt in Corynebacterium glutamicum DM 274-2 unter der Nummer DSM 4241) dar oder auch pCV34, pCV36, pCVX4, pCVX10, pCVX15, pZ9 und pZ8-1 (DE-OS 3841 454.6) oder pCV35, pECM3, pECM1 (DE-OS 3841 453.8).
Verwendbar sind aber auch die aus der EP-A-93 611 bekannten zusammengesetzten Plasmide, soweit sie in Corynebakterien oder Brevibakterien selbst replizieren, insbesondere pAJ 655, pAJ 611, pAJ 440, pAJ 1844 und pAJ 3148 aber auch pCG 11, pCE 54 (s. EP-A 0 233 581), ebenso pUL330 (Santamaria, R.I. et al., J. Bacteriology 162 (1985) 463-467).

Gegenstand der Anmeldung ist ebenso die Verwendung rekombinante DNA enthaltenden Mikroorganismen der Gattungen Corynebacterium oder Brevibacterium zur Herstellung von L-Lysin durch Fermentation.

Das klonierte DNA-Fragment (s. Abb. 2) enthält nur einen Bruchteil des Aspartat-Kinase Gens (lysC) sowie das vollständige Gen der Aspartyl-β-semialdehyd-Dehydrogenase (asd), wie aus der Sequenzanalyse erkennbar ist.

Der Bruchteil dieses Aspartat-Kinase Gens besitzt eine zur β-Untereinheit der Aspartat-Kinase II aus B.subtilis homologe DNA-Sequenz.

Alle Tranformanten, deren Plasmid diese Sequenz aufweist (pCS2, pCS21, pCS22, pCS23, pCS24, pCS26, pCS233), enthalten eine verglichen mit dem chromosomal codierten Enzym aus ATCC 13032 bezüglich der feed-back Inhibitoren L-Lysin und L-Threonin deutlich desensibilisierte Aspartatkinase und zeigen AEC-Resistenz.

Die aus Homologievergleichen gezogenen Schlüsse nach denen das Pst I - XhoI Genfragment aus DM58-1 nur ein Teil des lysC Gens (AK), aber das vollständige asd-Gen beherbergt, konnten durch Enzymmessungen eindeutig bestätigt werden.

Kein mit pCS2 oder einem pCS2-Derivat transformierter C.glutamicum ATCC13032 Stamm enthält überraschenderweise eine gegenüber dem Empfängerstamm erhöhte Aspartat-Kinase Aktivität (Tabelle 4, Spalte 3).

Demgegenüber ist in allen Transformanten, deren Plasmide das asd-Strukturgen enthalten, eine starke Überexpression der Aspartyl-β-semialdehyd-Dehydrogenase (ASA-DH) nachweisbar (Tabelle 4, Spalte 2, Abb. 3 und 4). Die Plasmide pCS23 und pCS23-Derivate führend erwartungsgemäß nicht zu einer Überexpression der ASA-DH.

Die bei Klonierung des erfindungsgemäßen DNA-Fragments mit Hilfe von pCS 2 und daraus abgeleiteten Derivaten eintretende starke Überexpression der ASA-DH gewährleistet eine effiziente Umsetzung des Produkts der Aspartat-Kinase Reaktion, dem β-Aspartylphosphat, wodurch eine Beschleunigung der nicht mehr inhibierbaren Aspartat-Kinase Reaktion eintritt.

Aufgrund der hohen Labilität der ASA-DH schwanken die Faktoren der aus der spezifischen Aktivität kalkulierbaren Überexpression von 31 - 65.

Gegenüber dem Stand der Technik ergibt sich eine wesentliche Vereinfachung daraus, daß erstens nur ein Bruchteil des lysC Gens, der zu einer Deregulation der Aspartat-Kinase führt, isoliert werden muß, um eine Lysin-Ausscheidung zu bewirken oder zu verbessern, und zweitens aufgrund der Organisation von lysC und asd in einem Operon so dass, das asd-Gen ohne zusätzlichen exp. Aufwand aufgrund der mit dem mutierten lysC-Gen auftretenden AEC-Resistenz zusammen mit dem lysC-Gen isoliert werden kann.Umgekehrt kann mit Hilfe von asd-Mutanten das lysC + asd enthaltende DNA-Fragment isoliert werden und zwar unabhängig davon, ob lysC mutiert ist oder nicht.

### 1. Charakterisierung des Genspenders DM58-1 und Genempfängers ATCC13032

### 1.1 Entwicklung und Phänotyp des Stammes DM58-1

Der Stamm DM58-1 wurde durch Mutagenese von Corynebacterium glutamicum Stamm ATCC13032 mit einer üblichen Konzentration an Ethylmethansulfonat entwickelt.
Die Selektion erfolgte durch Ausplattieren des so erhaltenen Mutantengemischs auf Minimal-Agar der Zusammensetzung 20 g Glucose; 10 g (NH₄)₂SO₄; 2,5 g Harnstoff; 1 g KH₂PO₄; 0,4 g MgSO₄·7H₂O; 2 mg FeSO₄·7H₂O; 1,5 mg MnSO₄·H₂O; 300 »g Biotin; 900 »g Thiamin und 20 g Agar pro 1 Aquadest (pH 7,0), der eine geeignete Konzentration an 5-2-Aminoethyl-D,L-Cystein (AEC) enthielt. Ein auf diesem Medium teilungsfähiger von einem solchen Selektionsmedium isolierter Klon, später als DM58-1 bezeichnet, trägt neben seiner AEC-Resistenz keine weiteren genetischen Marken.

### 1.2 Enzymgehalte an Aspartat-Kinase und Aspartyl-beta-semialdehyd Dehydrogenase in ATCC13032 und DM58-1

Die Stämme ATCC13032 und DM58-1 wurden unter direkt vergleichbaren Bedingungen in Standard I Bouillon (Merck Art. Nr. 7882) mit zusätzlichen 4 g/l Glucose und 1 mM MgCl₂ bei 30°C und 150 rpm bis zum Erreichen der früh stationären Phase kultiviert und durch Zentrifugation vom Kulturmedium getrennt. Man wäscht 3 Mal mit 100 mM Tris/HCl (pH 7,5); 1 mM DTT und suspendiert die Feuchtzellmasse in einem Volumenteil des gleiche Puffers.

Die so suspendierten Zellen wurden in einer Kugelmühle (B. Braun Melsungen - MSK-Homogenisator, IMA-Disintegrator S) durch Verrühren mit einer geeigneten Menge an Glasperlen aufgeschlossen. Das Zellhomogenat wurde mittels Glasfilternutsche von den Glasperlen getrennt und 30 Minuten bei 30000 x g klarzentrifugiert.

Nach 15stündiger Dialyse in Enzym-stabilisierendem Puffer wurden die Enzymaktivitäten in folgenden Testgemischen bestimmt:
Aspartat-Kinase Test: 100 mM Tris/HCl (pH 7,5), 1 mM DTT, 400 mM (NH₄)₂SO₄, 20 mM MgCl₂, 400 mM NH₂OH·HCl, 300 mM L-Aspartat, 40 mM ATP und verschiedene Mengen Enzympräparation.
Durch Zugabe von 750 »l einer Lösung aus 10 % Fe Cl₃ 6H₂O; 3,3 % TCA; 0,7 N HCl zu 500 »l des Enzymtestgemisches wird die Enzymreaktion nach 30 minütiger Inkubation bei 37°C gestoppt. Aus der mittels Eichkurvenverfahren photometrisch (ΔE_{540 nm}) bestimmten Aspartyl-beta-Hydroxamat Konzentration wird die in »Mol/mg·min (U/mg) angegebenen Enzymaktivität kalkuliert. Die zugehörigen Proteinkonzentrationen wurden nach der Methode von Lowry et al. (Lowry et al. J. Biol. Chem. 193, 265 (1951)) oder Bradford (Bradford Anal. Biochem. 72, 248 (1976)) durchgeführt.
Der Aspartyl-β-semialdehyd Dehydrogenase Test enthält 120 mM Diethanolamin (pH 9,0); 40 mM Na AsO₄; 1 mM NADP⁺; 5 mM L-Threonin; 1,3 mM Aspartyl-beta-semialdehyd und verschiedene Mengen Enzympräparation in einem Gesamtvolumen von 1 ml. Die in »Mol/mg·min (U/mg) angegebene Aktivitat wird über die photometrisch (ΔE_{540 nm}) bestimmte NADPH Synthesegeschwindigkeit berechnet.

Tabelle 1 enthält die spezifischen Enzymaktivitäten beider Enzyme in Rohextrakten identisch gezogener und aufgearbeiteter Zellen von C. glutamicum ATCC13032 und DM58-1. Neben vergleichbaren Gehalten an Aspartat-Kinase beider Stamme enthält die AEC resistente Mutante DM58-1 im Vergleich zum Wildtyp ca. 5fach erhöhte Aspartyl-β-semialdehyd Dehydrogenase Aktivität.

### 1.3 In vitro Hemmbarkeit der Aspartat-Kinase aus C. glutamicum ATCC13032 und DM58-1

Tabelle 1 zeigt, das die bereits von K. Nakayama et al. (K. Nakayama et al. Agr. Biol. Chem. 30, 611 (1966)) angedeutete und von S.N. Kara-Murza et al. (S.N. Kara-Murza Prikladnaya Biokhimiya; Mikrobiologia 14, 345 (1978)) genauer untersuchte Hemmbarkeit des C. glutamicum Wildtyp-Enzyms durch uns reproduziert werden konnte. Dem gegenübergestellt ist der deutlich differente Charakter des Enzyms der AEC resistenten Mutante DM58-1, deren Aspartat-Kinase nicht mehr konzertiert durch L-Lysin + L-Threonin hemmbar ist. Durch die am Enzym aus ATCC13032 Lysin-analog wirkenden Substanzen S-Aminoethyl-D,L-Cystein (AEC) wird das Enzym der Mutanten ebenfalls nur noch gering beeinflußt.

**Tabelle 1**

| Enzymgehalt und Eigenschaften von Aspartat-Kinase (AK) und Aspartyl-β-semialdehyd Dehydrogenase (ASA-DH) aus C. glutamicum ATCC13032 und DM58-1) | | |
|---|---|---|
| Stamm | ATCC13032 | DM58-1 |
| AK (U/mg) | 0,016 | 0,011 |
| ASA-DH (U/mg) | 0,06 | 0,33 |

| Hemmstoff-Kombinationen | AK-Hemmung (%) | |
|---|---|---|
| 10 mM L-Lys | 89 | 12 |
| 1 mM L-Thr | | |
| 10 mM L-Lys | 95 | 2 |
| 10 mM L-Thr | | |
| 100 mM L-Lys | 99 | 21 |
| 10 mM L-Thr | | |
| 10 mM AEC | 12 | 0 |
| 1 mM L-Thr | | |
| 10 mM AEC | 41 | 0 |
| 10 mM L-Thr | | |
| 100 mM AEC | 95 | 7 |
| 10 mM L-Thr | | |
| Abkürzungen: AEC S-(Aminoethyl)-D,L-Cystein | | |

### 2. Klonierung eine DNA-Fragments von C. glutamicum Stamm DM58-1, das für eine feed-back resistente Aspartat-Kinase kodiert.

### 2.1 Klonierung

Gesamt-DNA wurde aus C. glutamicum Stamm DM58-1, wie bei Chater et al. (Chater et al. Curr. Topics Microb. Immunol. 96, 69 (1982)) beschrieben, isoliert und partiell mit dem Restriktionsenzym PstI verdaut. Der vektor pZ1 (Abb. 1), der in der Deutschen Patentanmeldung 3737729.9 beschrieben ist, wurde mit PstI linearisiert und durch Behandlung mit alkalischer Phosphatase dephosphoryliert. Vektor-DNA und DM58-1 DNA wurden gemischt und mit T4 DNA-Ligase, wie bei Maniatis et al. (Maniatis, T et al. Molecular Cloning, A Laboratory Manual, Cold Spring Harbour Laboratory 1982) beschrieben, behandelt.
Die Transformation von C. glutamicum ATCC13032 mit dem Ligationsgemisch erfolgte, wie bei Thierbach et al. (Thierbach G. et al. Applied Microbiology and Biotechnology 29, 356 (1988)) beschrieben.

### Abbildung 1: Restriktionskarte des Plasmids pZ1. Der dick gezeichnete Strich stellt den pHM1519-Anteil, und der dünn gezeichnete Strich stellt den pACYC177-Anteil von pZ1 dar. Ap^{R}: Ampicillin-Resistenzgen; Km^{R}: Kanamycin-Resistenzgen.

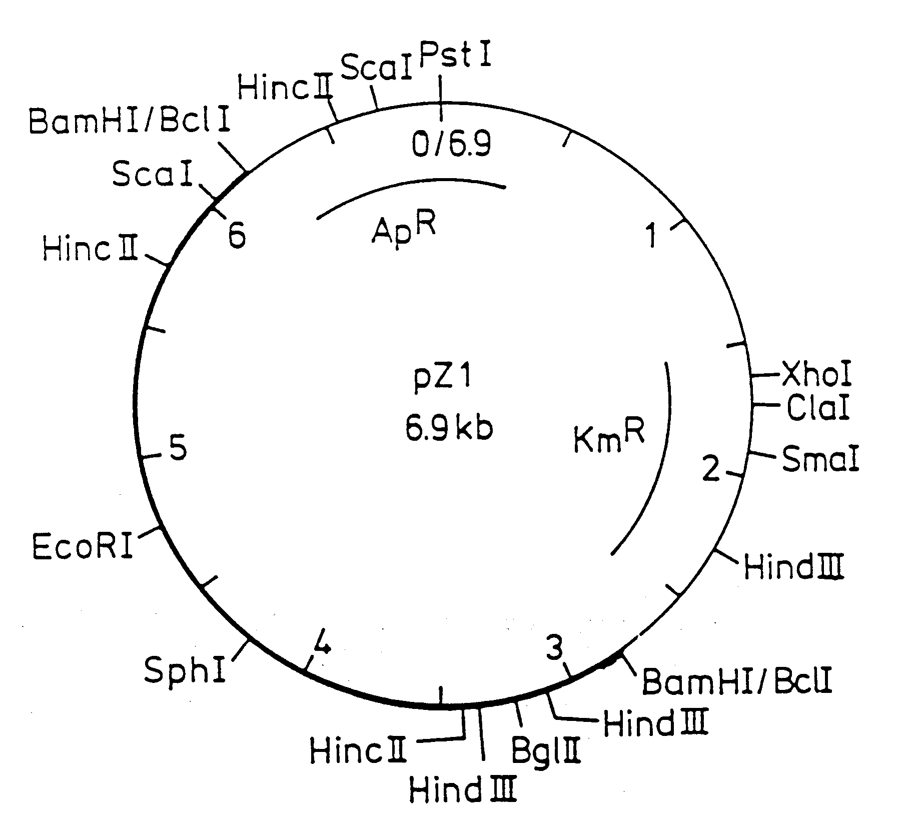
ScaI Pst I
HincII
BamHI/BclI
0/6.9
ScaI
6
ApR
1
HincII
pZ1
6.9 kb
XhoI
ClaI
KmR
SmaI
2
5
EcoRI
HindIII
3
BamHI/BclI
4
SphI
HindIII
BglII
HincII
HindIII

Das Transformationsgemisch wurde auf RCG/E-Agar mit 300 »g/ml Kanamycin ausplattiert und die Agarplatten eine Woche bei 30°C inkubiert. Anschließend wurden die Agarplatten auf MM-Agar (Katsumata, R. et al. J. Bact. 159, 306 (1984)) mit 50 mM AEC und 50 mM L-Threonin übergestempelt und einen Tag bei 30°C bebrütet. Eine Kolonie, die auf diesem Agar wachsen konnte wurde auf MM-Agar, der zusätzlich AEG, L-Threonin und 10 »g/ml Kanamycin enthielt, ausgestrichen, um Einzelkolonien zu erhalten. Plasmid DNA wurde aus einem derartigen Klon isoliert, als pCS2 bezeichnet und zur Transformation von C. glutamicum ATCC13032 verwendet. 59 von 62 überprüften Kanamycin-resistenten Transformanten erwiesen sich als resistent gegenüber der Hemmung durch 50 mM AEC und 50 mM L-Threonin. Das Plasmid pCS2 wurde weiterhin durch Restriktionskartierung charakterisiert. Es enthält eine ca. 9,9 kb lange Insertion in der PstI-Schnittstelle des vektors pZ1, der eine Länge von 6,9 kb hat. Die Restriktionskarte von pCS2 ist in Abbildung 2 dargestellt.

### Abbildung 2: Restriktionskarte des Plasmids pCS2 in linearisierter Form.

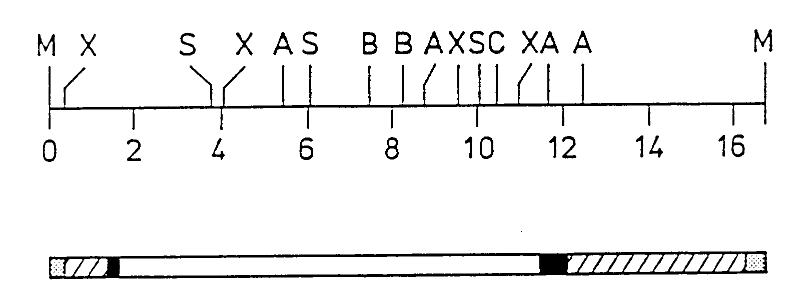
M X S X A S B B A XSC XA A M
0 2 4 6 8 10 12 14 16

Der obere Teil der Figur gibt die Position der verschiedenen Restriktionsschnittstellen wieder. Im unteren Teil der Figur sind verschiedene Regionen von Plasmid pCS2 dargestellt. Die Insertionen von DM58-1 DNA ist als offener Balken dargestellt. Das Ampicillin-Resistenzgen von pZ1 ist schwarz hervorgehoben, das Kanamycin-Resistenzgen ist durch Punktierung gekennzeichnet. Die übrigen pZ1-Anteile von pCS2 sind durch Schraffur hervorgehoben. Abkürzungen: BamHI, B; BclI, C; SalI, S; Sca, A; SmaI, M; XhoI, X.

### 2.2 Charakterisierung der Aspartat-Kinase-Aktivität

Aspartat-Kinase-Aktivität wurde in Stamm ATCC13032/pCS2, als positive Kontrolle in Stamm DM58-1 und als negative Kontrolle in Stamm ATCC13032 gemessen. Die Stämme wurden in Standard I Bouillon, das mit 4 g/l Glucose, 10 »g/ml Kanamycin und 1 mM MgCl₂ supplementiert war, kultiviert. Kulturbedingungen, Zellernte, Zellaufschluß und Bestimmung der Aspartat-Kinase wurden, wie unter 1.2 beschrieben, durchgeführt. Die Effektoren L-Lys, L-Thr und AEC werden jeweils als Stammlösungen in 100 mM Tris/HCl Puffer mit einem pH von 7,5 zugegeben.

Aspartat-Kinase-Gehalt und Hemmbarkeit des Enzyms aus ATCC13032/pCS2 sind in Tabelle 4 dargestellt. Obwohl der Stamm keine erhöhte spezifische Aktivität zeigte, konnte eine deutliche Desensibilisierung gegenüber den genannten Hemmstoffen nachgewiesen werden, deren Ausmaß den Grad der Deregulation des Enzyms aus dem Genspender DM58-1 allerdings nicht erreicht (partielle Deregulation).

### 2.3 Bestimmung der L-Lysin-Ausscheidung

Die Fähigkeit, Lysin auszuscheiden, wurde in Stamm ATCC13032/pCS2 und als negative Kontrolle in Stamm ATCC13032/pZ1 bestimmt. Nach Zusatz von 10 »g/ml Kanamycin wurde die Kultur, wie unten beschrieben, durchgeführt. Das Ergebnis des Versuches ist in Tabelle 2 zusammengefaßt.

**Tabelle 2**

| Ausscheidung von L-Lysin durch verschiedene C. glutamicum Stämme. | |
|---|---|
| C. glutamicum Stamm | Konzentration an ausgeschiedenem L-Lysin·HCl (g/l) |
| ATCC13032/pZ1 | 0,0 |
| ATCC13032/pCS2 (≙ DM 2-1/pCS2) | 7,1 |

Ein 100 ml Erlenmeyerkolben mit Schikanen wird dabei mit 10 ml des folgenden Kulturmediums befüllt: 12 g/l Ammoniumsulfat, 240 g/l Melasse, 60 ml/l Sojamehlhydrolysat und 10 g/l CaCO₃. Nach Animpfen werden die Kulturen 72 Stunden bei 30_{°}C und 300 rpm inkubiert. Die Lysin-Bestimmung erfolgte im zentrifugiertan Überstand mit Hilfe von Aminosäureanalysatoren.

### 3. Deletionskartierung des DNA-Fragments von pCS2, das für eine feed-back resistente Aspartat-Kinase kodiert.

Durch vollständige oder partielle Verdauung von pCS2 mit verschiedenen Restriktionsenzymen und anschließender Behandlung mit T4 DNA-Ligase bei niedriger DNA Konzentration wurden verschiedene Deletionsderivate konstruiert. Die Herstellung der verschiedenen Deletionsderivate ist in Tabelle 3 zusammengefaßt und die Position der Deletionen in den verschiedenen Derivaten in Abbildung 3 dargestellt. In Abbildung 3 ist ebenfalls das Resistenzverhalten der von C. glutamicum ATCC13032 abgeleiteten Stamme gegenüber AEC eingetragen. Auf diese Weise konnte die AEC-Resistenz vermittelnde DNA-Region auf ein ca. 1,5 Kb langes DNA Fragment eingegrenzt werden, welches in Plasmid pCS233 durch die PstI-Klonierschnittstelle und eine EcoRI-Schnittstelle begrenzt wird.

Die Aspartat-Kinase-Aktivität und die Hemmbarkeit der Enzymaktivität durch Mischungen von Lysin bzw. AEC und Threonin wurde in den konstruierten Klonen bestimmt. Anzucht, Aufschluß und Aktivitätsbestimmung wurden, wie oben beschrieben, durchgeführt. Außerdem wurde die Fähigkeit der verschiedenen Klone untersucht, L-Lysin auszuscheiden. Hierfür wurde ein Agarplatten-Diffusionstest mit einem L-Lysin-auxotrophen Indikationsstamm von C. glutamicum verwendet. Aus Tabelle 4 ist zu entnehmen, daß sämtliche AEC resistenten Stämme eine partiell deregulierte Aspartat-Kinase-Aktivität besitzen und in der Lage sind, L-Lysin auszuscheiden.

**Tabelle 3**

| Herstellung und AEC^{R/S}-Phänotyp verschiedener Deletionsderivate des Plasmids pCS2 | | |
|---|---|---|
| Plasmid | Konstruktion | AEC^{R/S}-Phänotyp |
| pCS21 | hergestellt nach Verdauung von pCS2 mit BamHI | R |
| pCS22 | hergestellt nach Verdauung von pCS2 mit BamHI und BclI | R |
| pCS23 | hergestellt nach partieller Verdauung von pCS2 mit SalI | R |
| pCS24 | hergestellt nach partieller Verdauung von pCS2 mit XhoI | R |
| pCS26 | hergestellt nach Verdauung von pCS2 mit ScaI | R |
| pCS231 | hergestellt nach partieller Verdauung von pCS23 mit PstI | S |
| pCS232 | hergestellt nach partieller Verdauung von pCS23 mit DraI | S |
| pCS233 | hergestellt nach partieller Verdauung von pCS23 mit EcoRI | R |
| Zeichenerklärung: R = Resistenz, S = Sensitivität | | |

### Abbildung 3: Deletionskarte des Plasmids pCS2

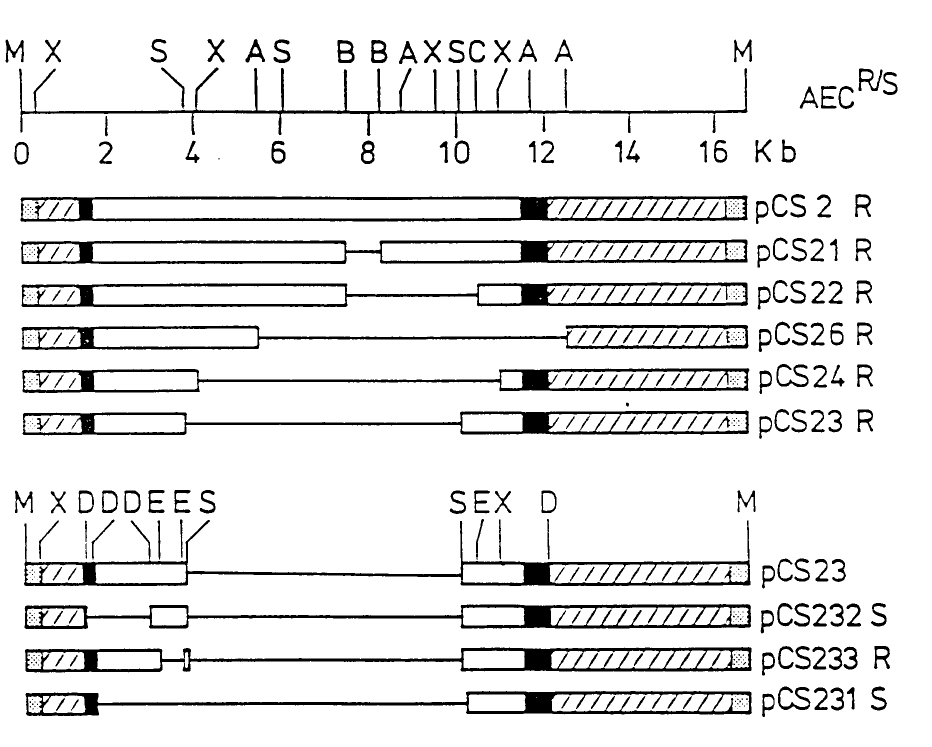
M X S X A S B B A X S C X A A M
AEC^R/S
0 2 4 6 8 10 12 14 16 Kb
 pCS 2 R
 pCS21 R
 pCS22 R
 pCS26 R
 pCS24 R
 pCS23 R
M X D D D E E S S E X D M
 pCS23
 pCS232 S
 pCS233 R
 pCS231 S

Der obere Teil der Figur zeigt die von pCS2 abgeleiteten Derivate, der untere Teil zeigt die von pCS23 abgeleiteten Derivate. Das Ampicillin Resistenzgen von pZ1 ist schwarz hervorgehoben; das Kanamycin Resistenzgen ist gepunktet dargestellt; die übrigen pZ1 Anteile von pCS2 sind durch Schraffur gekennzeichnet. Die Insertion von DM58-1 DNA ist als offener Balken dargestellt. Die Deletionen sind als Strich gekennzeichnet. Abkürzungen: BamHI, B; BclI C; DraI, D; EcoRI, E; SalI, S; ScaI, A; SmaI, M; XhoI, X:

**Tabelle 4**

| Mikrobiologische und biochemische Charakterisierung rekombinanter C. glutamicum Stämme | | | | | | | | |
|---|---|---|---|---|---|---|---|---|
| Stamm | ASA-DH (U/mg) | AK (U/mg) | AK Restaktivität (%) in Anwesenheit von | | | | AEC^{R} | Lysin-Ausscheidung |
| | | | 10mM Lys 1mM Thr | 10mM Lys 10mM Thr | 100mM Lys 10mM Thr | 100mM AEC 10mM Thr | | |
| ATCC13032 (pZ1) | 0,06 | 0,016 | 9 | 5 | 3 | 7 | - | - |
| ATCC13032 (pCS2) | 3,9 | 0,013 | 55 | 55 | 28 | 56 | + | + |
| ATCC13032 (pCS21) | n.b. | 0,016 | 46 | 50 | 24 | n.b. | + | + |
| ATCC13032 pCS22 | n.b. | 0,015 | 40 | 41 | 22 | n.b. | + | + |
| ATCC13032 (pCS23) | 0,03 | 0,014 | 51 | 57 | 30 | 56 | + | + |
| ATCC13032 (pCS24) | 2,07 | 0.011 | 65 | 65 | 40 | 62 | + | + |
| ATCC13032 (pCS26) | 1,88 | 0,015 | 64 | 63 | 39 | 60 | + | + |
| ATCC13032 (pCS231) | 0,060 | 0,013 | 11 | 14 | 4 | 10 | - | - |
| ATCC13032 (pCS232) | n.b. | n.b. | n.b. | n.b. | n.b. | n.b. | - | n.b. |
| ATCC13032 (pCS233) | n.b. | 0,011 | 56 | 62 | 36 | 57 | + | n.b. |
| DM58-1 (pZ1) | 0,330 | 0,009 | 83 | 100 | 79 | 93 | + | + |
| Abkürzungen: ASA-DH: Aspartyl-β-semialdehyd Dehydrogenase AK : Aspartat-Kinase n.b. : nicht bestimmt | | | | | | | | |

### 4. Sequenzierung eines DNA Fragments von Plasmid pCS24, welches den Phänotyp AEC-Resistenz vermittelt.

### 4.1 Sequenziermethode

Die Nukleotidsequenz des 2.1 Kb PstI-XhoI-DNA-Fragments wurde nach der Methode von Maxam und Gilbert (Maxam, A.M. et al., Proc. Natl. Acad. Sci. USA 74, 560-564 (1977)) mit den Modifikationen von Arnold und Pühler (Arnold, W. et al., Gene, 70,171 ff (1988)) bestimmt. Die Subklonierung zur Sequenzierung ging dabei vom Plasmid pCS24 aus (Abb. 4). Dieses wurde nach E. coli MM 294 (Meselson, M. et al., Nature 217, 1110-1114 (1968)) transformiert und entsprechende Fragmente in die Sequenziervektoren pSVB21, 25 und 26 (Arnold, W. et al., Gene, 70,171 ff (1988)) kloniert. Im E. coli-Stamm JM83 (Messing, J. Recombinant DNA Technical Bulletin NIH Publication No. 79-99 2, 43-48 (1979)) konnte Insertionsinaktivierung mittels XGal-Test (5-Bromo-4-chloro-indolyl-β-D-galactopyranosid) nachgewiesen werden.

Die Sequenzierstrategie ist in Abb. 4 wiedergegeben. Die Nukleotidsequenz wurde von beiden DNA-Strängen mit überlappenden Klonen ermittelt.

### 4.2 DNA-Sequenz des 2.1 Kb Pst I- Xho I - DNA Fragments

Das sequenzierte DNA-Stück ist 2112 bp lang. Es trägt Restriktionsschnittstellen für die Enzyme BglII, DraI, EcoRI, HindIII, NaeI, PstI, SalI und XhoI, mit denen auch die Subklone hergestellt wurden (Abb. 5).
Die Nukleotidsequenz wurde mit dem Sequenzanalyse-Programmpaket ANALYSEQ (Staden, R. et al., Nucl. Acids Res. 14, 217-232 (1986)) bearbeitet.

### Abb. 4 Deletionsanalyse und Sequenzierstrategie des chromosomalen Fragments des Plasmids pCS2.

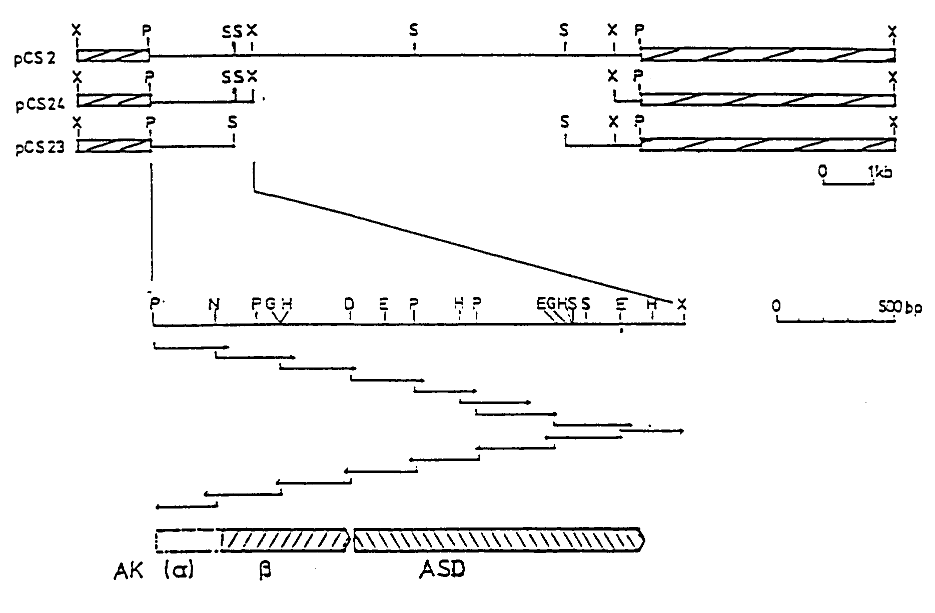
X P SS X S S X P X
pCS2
X P SS X X P X
pCS24
X P S S X P X
pCS23
0 1kb
P N P G H D E P H P EGHS S E H X
0 500bp
AK (α) β ASD

Deletionsanalyse: die Plasmide pCS23 und pCS24 vermitteln ebenso wie der Klon pCS2 AEC-Resistenz und Lysin-Produktion. Die schraffierten Balken stellen den Vektoranteil der Plasmide dar.
Sequenzierstrategie: das 2.1 kb *Pst*I-*Xho*I-Fragment des Plasmids pCS24 wurde mit den eingezeichneten Restriktionsschnittstellen subkloniert. Die Pfeile geben jeweils den sequenzierten Bereich und die Leserichtung an. Eingezeichnet sind die Restriktionsschnittstellen der Enzyme *Dra*I (D), *Eco*RI (E), *Bgl*II (G), *Hind*III (H), *Nae*I (N), *Pst*I (P), *Sal*I (S) und *Xho*I (X). Darunter sind die 2 offenen Leseraster gezeigt, die für die Untereinheiten der Aspartatkinase und für Aspartat-β-Semialdehyd-Dehydrogenase codieren (s. Text).

Es finden sich 2 lange offene Leseraster (ORF) auf dem sequenzierten DNA-Stück. Beide sind von der PstI-Schnittstelle zur XhoI-Schnittstelle hin angeordnet. Es befindet sich nur ein kleiner Bereich von 26 bp zwischen beiden. Vor dem 2. ORF befindet sich eine Ribosomenbindungsstelle (RBS) (806-809 AGGA gefolgt von dem Startcoon ATG). Ebenso wurde innerhalb des 1. ORF's eine RBS lokalisiert (AGGA, 268-271 mit Startcodon GTG).
Der ORF1 hat eine Länge von 264 Aminosäuren (AS), gerechnet von der PstI-Stelle und von 172 AS (entsprechend 18,6 k Dals) von der internen RBS aus. ORF2 ist 342 AS (36,1 K Dals) lang.
Direkt hinter dem ORF2 befindet sich eine mögliche Transkriptionsterminationsstruktur, eine sogenannte Haarnadelschleife, gefolgt von mehreren Thyminresten (1864-1900). Diese Anordnung ist charakteristisch für ρ-unabhängige Terminationssignale in E. coli und anderen Bakterienspezies (Ahyda et al. Ann. Rev. Biochem. 47, 967-996 (1978)). Der hier vorliegende Terminator hat eine Stabilität von mehr als -40 kcal/mol bei 30°C.

Ein möglicher Promoter für ORF2 wurde innerhalb des ORF1 ermittelt (409-437), TTGACA-17 bp-TATTCT). Die -35-Region und der Abstand zur -10-Region entsprechen genau dem E. coli-Consensus-Promotor (Hawley, D.K. et al., Nucl. Acids Res. 11, 2237-2255 (1983)), die -10-Region ist der E. coli-Consensus-region (TATAAT) sehr ähnlich.

Abb.5 DNA-Sequenz und abgeleitete Aminosäuresequenzen des 2.1 kb PstI-XhoI-Fragments

AlaValAlaLeuAlaAlaAlaLeuAsnAlaAspValCysGluIleTyrSerAspValAspGlyValTyrThrAlaAspProArgIleV
CTGCAGTTGCGTTGGCAGCTGCTTTGAACGCTGATGTGTGTGAGATTTACTCGGACGTTGACGGTGTGTATACCGCTGACCCGCGCATCG
PstI 10 20 30 40 50 60 70 80 90

ProAsnAlaGlnLysLeuGluLysLeuSerPheGluGluMetLeuGluLeuAlaAlaValGlySerLysIleLeuValLeuArgSerV
TTCCTAATGCACAGAAGCTGGAAAAGCTCAGCTTCGAAGAAATGCTGGAACTTGCTGCTGTTGGCTCCAAGATTTTGGTGCTGCGCAGTG
100 110 120 130 140 150 160 170 180

GluTyrAlaArgAlaPheAsnValProLeuArgValArgSerSerTyrSerAsnAspProGlyThrLeuIleAlaGlySerMetGluA
TTGAATACGCTCGTGCATTCAATGTGCCACTTCGCGTACGCTCGTCTTATAGTAATGATCCCGGCACTTTGATTGCCGGCTCTATGGAGG
190 200 210 220 230 240 250 NaeI ***

IleProValGluGluAlaValLeuThrGlyValAlaThrAspLysSerGluAlaLysValThrValLeuGlyIleSerAspLysProG
ATATTCCTGTGGAAGAAGCAGTCCTTACCGGTGTCGCAACCGACAAGTCCGAAGCCAAAGTAACCGTTCTGGGTATTTCCGATAAGCCAG
* --> 290 300 310 320 330 340 350 360

GluAlaAlaLysValPheArgAlaLeuAlaAspAlaGluIleAsnIleAspMetValLeuGlnAsnValTyrSerValGluAspGlyT
GCGAGGCTGCGAAGGTTTTCCGTGCGTTGGCTGATGCAGAAATCAACATTGACATGGTTCTGCAGAACGTCTATTCTGTAGAAGACGGCA
370 380 390 400 410 420 PstI 430 440 450

ThrAspIleThrPheThrCysProArgSerAspGlyArgArgAlaMetGluIleLeuLysLysLeuGlnValGlnGlyAsnTrpThrA
CCACCGACATCACCTTCACCTGCCCTCGTTCCGACGGCCGCCGCGCGATGGAGATCTTGAAGAAGCTTCAGGTTCAGGGCAACTGGACCA
460 470 480 490 500 BglII 510 HindIII 530 540

ValLeuTyrAspAspGlnValGlyLysValSerLeuValGlyAlaGlyMetLysSerHisProGlyValThrAlaGluPheMetGluA
ATGTGCTTTACGACGACCAGGTCGGCAAAGTCTCCCTCGTGGGTGCTGGCATGAAGTCTCACCCAGGTGTTACCGCAGAGTTCATGGAAG
550 560 570 580 590 600 610 620 630

LeuArgAspValAsnValAsnIleGluLeuIleSerThrSerGluIleArgIleSerValLeuIleArgGluAspAspLeuAspAlaA.
CTCTGCGCGATGTCAACGTGAACATCGAATTGATTTCCACCTCTGAGATTCGTATTTCCGTGCTGATCCGTGAAGATGATCTGGATGCTG
640 650 660 670 680 690 700 710 720

AlaArgAlaLeuHisGluGlnPheGlnLeuGlyGlyGluAspGluAlaValValTyrAlaGlyThrGlyArgOC
CTGCACGTGCATTGCATGAGCAGTTCCAGCTGGGCGGCGAAGACGAAGCCGTCGTTTATGCAGGCACCGGACGCTAAAGTTTTAAAGGAG
730 740 750 760 770 780 790 800 DraI****

MetThrThrIleAlaValValGlyAlaProAlaArgSerAlaArgLeuCysAlaProPheTrpLysSerAlaIleSerGl
TAGTTTTACAATGACCACCATCGCAGTTGTTGGTGCACCGGCCAGGTCGGCCAGGTTATGCGCACCCTTTTGGAAGAGCGCAATTTCCCA
820--> 830 840 850 860 870 880 890 900

LeuThrLeuPheValSerLeuLeuProThrSerAlaGlyArgLysIleGluPheArgGlyThrGluIleGluValGluAspIleThrGl
GCTGACACTGTTCGTTTCTTTGCTCCCCACGTCCGCAGGCCGTAAGATTGAATTCCGTGGCACGGAAATCGAGGTAGAAGACATTACTCA
910 920 930 940 950 EcoRI 960 970 980 990

AlaThrGluGluSerLeuLysAspIleAspValAlaLeuPheSerAlaGlyGlyThrAlaSerLysGlnTyrAlaProLeuPheAlaAl
GGCAACCGAGGAGTCCCTCAAGGACATCGACGTTGCGTTGTTCTCCGCTGGAGGCACCGCTTCCAAGCAGTACGCTCCACTGTTCGCTGC
1000 1010 1020 1030 1040 1050 1060 1070 PstI

AlaGlyAlaThrValValAspAsnSerSerAlaTrpArgLysAspAspGluValProLeuIleValSerGluValAsnProSerAspLy
TGCAGGCGCGACTGTTGTGGATAACTCTTCTGCTTGGCGCAAGGACGACGAGGTTCCACTAATCGTCTCTGAGGTGAACCCTTCCGACAA
1090 1100 1110 1120 1130 1140 1150 1160 1170

AspSerLeuValLysGlyIleIleAlaAsnProAsnCysThrThrMetAlaAlaMetProValLeuLysProLeuHisAspAlaAlaGl
GGATTCCCTGGTCAAGGGCATTATTGCGAACCCTAACTGCACCACCATGGCTGCGATGCCAGTGCTGAAGCCACTTCACGATGCCGCTGG
1180 1190 1200 1210 1220 1230 1240 1250 1260

LeuValLysLeuHisValSerSerTyrGlnAlaValSerGlySerGlyLeuAlaGlyValGluThrLeuAlaLysGlnValAlaAlaVa
TCTTGTAAAGCTTCACGTTTCCTCTTACCAGGCTGTTTCCGGTTCTGGTCTTGCAGGTGTGGAAACCTTGGCAAAGCAGGTTGCTGCAGT
HindIII 1280 1290 1300 1310 1320 1330 1340 PstI

GlyAspHisAsnValGluPheValHisAspGlyGlnAlaAlaAspAlaArgCysArgThrLeuCysPheThrAsnArgLeuGlnArgAl
TGGAGACCACAACGTTGAGTTCGTCCATGATGGACAGGCTGCTGACGCGCGATGTCGGACCTTATGTTTCACCAATCGCTTACAACGTGC
1360 1370 1380 1390 1400 1410 1420 1430 1440

AlaIleAlaGlyAsnLeuValAspAspGlyThrPheGluThrAspGluGlnLysLeuArgAsnGluSerArgLysIleLeuGlyLe
TGCCATCGCCGGAAACCTCGTCGATGACGGCACCTTCGAAACCGATGAAGAGCAGAAGCTGCGCAACGAATCCCGCAAGATTCTCGGTCT
1450 1460 1470 1480 1490 1500 1510 1520 1530

ProAspLeuLysValSerGlyThrCysValArgValProValPheThrGlyHisThrLeuThrIleHisAlaGluPheAspLysAlaIl
CCCAGACCTCAAGGTCTCAGGCACCTGCGTTCGCGTGCCGGTTTTCACCGGCCACACGCTGACCATTCACGCCGAATTCGACAAGGCAAT
1540 1550 1560 1570 1580 1590 1600 EcoRI 1620

ThrValAspGlnAlaGlnGluIleLeuGlyAlaAlaSerGlyValLysLeuValAspValProThrProLeuAlaAlaAlaGlyIleAs
CACCGTGGACCAGGCGCAGGAGATCTTGGGTGCCGCTTCAGGCGTCAAGCTTGTCGACGTCCCAACCCCACTTGCAGCTGCCGGCATTGA
1630 1640 BglII1650 1660 HindIII SalI 1690 1700 1710

GluSerLeuValGlyArgIleArgGlnAspSerThrValAspAspAsnArgGlyLeuValLeuValValSerGlyAspAsnLeuArgLy
CGAATCCCTCGTTGGACGCATCCGTCAGGACTCCACTGTCGACGATAACCGCGGTCTGGTTCTCGTCGTATCTGGCGACAACCTCCGCAA
1720 1730 1740 SalI 1760 1770 1780 1790 1800

AsnAlaAlaLeuAsnThrIleGlnIleAlaGluLeuLeuValLys
GAATGCTGCGCTAAACACCATCCAGATCGCTGAGCTGCTGGTTAAGTAAAAACCCGCCATTAAAAACTCCGCTTGAGTGCTACACTTTAA
1810 1820 1830 1840 1850 1860 1870 1880 1890

GCGGGGTTTTAATGTTTGAGGGGCGATGGGGGTCGAGCTTGTGAAGTGGAATTCTCCACAAGTTTTAAGTTTCTTTAGCAGGGGAAACAC
1900 1910 1920 1930 EcoRI 1950 1960 1970 1980

TGCTGATAGCGCTAGCGATAAAGAACATGAAAATGCAACGGAGCTAGCGGCCGAAGCTTTAGCGGATGTCATTTTTCAGTGGAAAAACTG
1990 2000 2010 2020 2030 HindIII 2050 2060 2070

GGTCTACCGACGCGTTGATAGTGTGCATCCATCCAGCTCGAG
2080 2090 2100 XhoI

Die Aminosäuresequenzen von ORF1 (1-794) und ORF2 (821-1846) sind im 3-Buchstaben-Code
angegeben. Die Nummerierung unterhalb des DNA-Stranges bezieht sich auf die DNA-Sequenz.
Die Namen der zur Sequenzierung benutzten Klonierschnittstellen sind ebenfalls unterhalb
eingetragen. Ribosomenbindungsstellen sind durch Sterne (*), Startcodons durch Pfeile
(-->) und die Terminatorstruktur durch Balken (———) markiert.

### 4.3 Analyse der Aminosäuresequenz

Die von ORF1 und ORF2 translatierten Aminosäuresequenzen wurden mit den bekannten Sequenzen der Aspartat-Kinasen (AK) I (Cassan, M. et al., J. Biol. Chem. 261, 1052-1057 (1986)) von E. coli und der AK II von B. subtilis (Chen, N.-Y. et al., J. Biol. Chem 262, 8737-2255 (1987)) bzw. den AS-Sequenzen der Aspartatsemialdehyd-Dehydrogenasen (ASA-DH) von E. coli (Haziza, C. et al., Embo J. 1, 379-384 (1982)) und Streptococcus mutans (Cardineau, G.A. et al., J. Biol. Chem. 262, 7, 3344-3353 (1987)) verglichen. Dazu wurden die Programme MALIGN (Sobel, E. et al., Nucl. Acids Res. 14, 363-374 (1986)) und DIAGON (Staden, R. et al. Nucl. Acid Res. 14, 217-232 (1986)) benutzt. Es zeigten sich signifikante Übereinstimmungen zwischen ORF1 und den AK-Sequenzen einerseits und ORF2 und der ASA-DH-Sequenz von S. mutans andererseits. Zur E. coli ASA-DH zeigten sich nur schwache Homologien, vornehmlich allerdings im Bereich des aktiven Zentrums (Haziza, C. et al., Embo J. 1, 379-384 (1982)).

Aus den Computeranalysen ergibt sich folgendes:
- ORF1 entspricht dem C. Terminus der Aspartat-Kinase, d.h. es fehlen etwa 160 AS vom N-Terminus sowie die komplette Promotorregion.
- ORF2 entspricht der Aspartatsemialdehyd-Dehydrogenase.

Die Homologie des ORF1 mit der B. subtilis-AK II ist für den Fachmann augenfällig. Die AK II besteht aus überlappenden Untereinheiten (Chen, N.-Y. et al., J. Biol. Chem 262, 8787-8798 (1987)). Dabei entspricht die β-Untereinheit dem C-Terminus der α-Untereinheit. Da die im ORF1 gefundene RBS in ihrer Position genau mit der RBS dieser AK übereinstimmt, kann im Analogieschluß gefolgert werden, daß hier die klonierte β-Untereinheit der AK von C. glutamicum vorliegt.

### 5. Expressionsexperimente

### 5.1 Komplementation asd- und lysC-negativer Stämme von E. coli.

Die Identität des ORF2 mit dem asd-Gen konnte durch Komplementation des asd-negativen E. coli-Stammes RASA 6 (Richaud, F. et al., C.R. Acad. Sc. Paris, 293, 507-512 (1981)) durch die Plasmide pCS2 und pCS24 belegt werden. pCS23, bei dem etwa 50 Aminosäuren vom C-Terminus der ASA-DH fehlen, komplementiert nicht. Keines dieser Plasmide war in der Lage, den AKI-III negativen E. coli Gif 106 M1 (Boy, E. et al., Biochemie 61, 1151-1160 (1979)) zu komplementieren.

### 5.2 Bestimmung der spezifischen Aspartat-Kinase (AK) und Aspartyl-β-semialdehyd-Dehydrogenase (ASA-DH) in Transformanten von ATCC13032 mit verschiedenen pCS2 Deletionsderivaten.

Die unter 4.3 aus Homologievergleichen gefolgerten Analogieschlüsse, nach denen das PstI-XhoI Genfragment aus DM58-1 nur ein Teil des lysC Gens (AK), aber das vollständige asd-Gen beherbergt, konnten durch Enzymmessungen eindeutig bestätigt werden.

Kein mit pCS2 oder einem pCS2 Derivat transformierter C. glutamicum ATCC13032 Stamm enthält eine gegenüber dem Empfängerstamm erhöhte Aspartat-Kinase Aktivität (Tabelle 4, Spalte 3).

Demgegenüber war in allen Transformanten, deren Plasmide das asd-Strukturgen enthielten, eine starke Überexpression der ASA-DH nachweisbar (Tabelle 4, Spalte 2, Abbildung 3 und 4). Die Plasmide pCS23 und pCS23-Derivate führten erwartungsgemäß nicht zu einer Überexpression der ASA-DH. Aufgrund der hohen Labilität der ASA-DH schwanken die Faktoren der aus der spezifischen Aktivität kalkulierbaren Überexpression von 31 - 65.

### 6. Enzymeigenschaften und L-Lysin Ausscheidung

Die für ATCC13032 pCS2 nachgewiesene L-Lysin Ausscheidung von 7,1 g/l in 72 Stunden (Tabelle 2) läßt sich damit auf zwei gentechnisch realisierte Veränderungen zurückführen.
a) Klonierung der Regulationsuntereinheit der Aspartat-Kinase aus DM58-1 ohne Erhöhung des zellulären Enzymgehaltes,
b) Klonierung der Aspartyl-β-semialdehyd-Dehydrogenase aus DM58-1, die zur 31-65fachen Erhöhung des zellulären Enzymgehalts führt.

## Patentansprüche (Patentansprüche für folgende(n) Vertragsstaat(en): AT, BE, DE, FR, GB, IT, NL)

1. DNA-Fragment, im wesentlichen bestehend aus einer genetischen Sequenz, mit der Länge von 2,1 kb, begrenzt durch eine Pst I- und eine Xho I-Schnittstelle,
gekennzeichnet durch die in Abb. 5 wiedergegebenen Aminosäuresequenz, die für die Produktion von Proteinen, die zu einer Aspartyl-β-semialdehyd-Dehydrogenase (asd) Aktivität und zur Deregulation der Aspartat-Kinase (lysC) führen, kodiert, enthalten in dem Plasmid pCS2, dessen Deletionskarte Abb. 3 zu entnehmen ist, hinterlegt in Corynebacterium glutamicum unter DSM 5086.

2. Aus dem Plasmid pCS2 gemäß Anspruch 1 abgeleitete rekombinante DNA mit den den Bezeichnungen pCS26 entsprechenden Deletionskarten, wiedergegeben in Abb. 3.

3. Aus dem Plasmid pCS2 gemäß Anspruch 1 abgeleitete rekombinante DNA mit den den Bezeichnungen pCS23 oder pCS223 entsprechenden Deletionskarten, wiedergegeben in Abb. 3

4. Verfahren zur Herstellung von L-Lysin durch Fermentation von diese Aminosäure ausscheidenden Mikroorganismen der Gattungen Corynebacterium oder Brevibacterium,
dadurch gekennzeichnet, daß die Mikroorganismen das Plasmid pCS2 gemäß Anspruch 1 oder eine davon abgeleitete rekombinante DNA gemäß den Ansprüchen 2 und 3 enthalten.

5. Verfahren gemäß Anspruch 4,
dadurch gekennzeichnet, daß man Corynebacterium glutamicum DSM 5086 einsetzt.

## Patentansprüche (Patentansprüche für folgende(n) Vertragsstaat(en): ES)

1. Verfahren zur Herstellung von L-Lysin durch Fermentation von diese Aminosäure ausscheidenden Mikroorganismen der Gattungen Corynebacterium oder Brevibacterium,
dadurch gekennzeichnet, daß die Mikroorganismen das Plasmid pCS2 enthalten, dessen Deletionskarte Abb. 3 zu entnehmen und das in Corynebacterium glutamicum unter DSM 5086 hinterlegt ist, enthaltend ein DNA-Fragment, im wesentlichen bestehnd aus einer genetischen Sequenz, mit der Länge von 2,1 kb, begrenzt durch eine Pst I- und eine Xho I-Schnittstelle,
gekennzeichnet durch die in Abb. 5 wiedergegebenen Aminosäuresequenz, die für die Produktion von Proteinen, die zu einer Aspartyl-β-semialdehyd-Dehydrogenase (asd) Aktivität und zur Deregulation der Aspartat-Kinase (lysC) führen, kodiert.

2. Verfahren gemäß Anspruch 1,
dadurch gekennzeichnet, daß die Mikroorganismen eine aus dem Plasmid pCS2 abgeleitete rekombinante DNA mit den der Bezeichnung pCS26 entsprechenden Deletionskarte, wiedergegeben in Abb. 3, enthalten.

3. Verfahren gemäß Anspruch 1,
dadurch gekennzeichnet, daß die Mikroorganismen eine aus dem Plasmid pCS2 abgeleitete rekombinante DNA mit den den Bezeichnungen pCS23 oder pCS223 entsprechenden Deletionskarten, wiedergegeben in Abb. 3, enthalten.

4. Verfahren gemäß Anspruch 1,
dadurch gekennzeichnet, daß man Corynebacterium glutamicum DSM 5086 einsetzt.

## Claims (Claims for the following Contracting State(s): AT, BE, DE, FR, GB, IT, NL)

1. DNA-fragment, consisting essentially of a genetic sequence, having a length of 2.1 kb, flanked by a Pst I and a Xho I cleavage site,
characterised by the amino acid sequence given in Figure 5, which sequence codifies the production of proteins giving rise to an aspartyl-β-semialdehyde dehydrogenase (asd) activity and to the deregulation of the aspartate-kinase (lysC), contained in the plasmid pCS2 the deletion map of which can be read in Figure 3, deposited in Corynebacterium glutamicum under DSM 5086.

2. Recombinant DNA derived from the plasmid pCS2 according to claim 1 and having the deletion maps corresponding to the notation pCS26 given in Figure 3.

3. Recombinant DNA derived from the plasmid pCS2 according to claim 1 and having the deletion maps corresponding to the notations pCS23 or pCS223 given in Figure 3.

4. A process for preparing L-lysin by fermentation of amino acid-carrying microorganisms of the genera Corynebacterium or Brevibacterium,
characterised in that the microorganisms contain the plasmid pCS2 according to claim 1 or a recombinant DNA derived therefrom according to claims 2 and 3.

5. A process according to claim 4,
characterised in that Corynebacterium glutamicum DSM 5086 is used.

## Claims (Claims for the following Contracting State(s): ES)

1. A process for preparing L-lysin by fermentation of amino-acid carrying microorganisms of the genera Corynebacterium or Brevibacterium,
characterised in that the microorganisms contain the plasmid pCS2, the deletion map of which can be read in Figure 3 and which is deposited in Corynebacterium glutamicum under DSM 5086, containing a DNA-fragment consisting essentially of a genetic sequence having a length of 2.1 kb, flanked by a Pst I and a Xho I cleavage site,
characterised by the amino acid sequence given in Figure 5, which sequence codifies the production of proteins giving rise to an aspartyl-β-semialdehyde-dehydrogenase (asd) activity and to the deregulation of the aspartate-kinase (lysC).

2. A process according to claim 1,
characterised in that the microorganisms contain a recombinant DNA derived from the plasmid pCS2 and having the deletion map corresponding to the notation pCS26 given in Figure 3.

3. A process according to claim 1,
characterised in that the microorganisms contain a recombinant DNA derived from the plasmid pCS2 and having the deletion maps corresponding to the notations pCS23 or pCS223 given in Figure 3.

4. A process according to claim 1,
characterised in that Corynebacterium glutamicum DSM 5086 is used.

## Revendications (Revendications pour l'(les) Etat(s) contractant(s) suivant(s): AT, BE, DE, FR, GB, IT, NL)

1. Fragment d'ADN qui consiste pour l'essentiel d'une séquence génétique ayant une longueur de 2,1 Kb limitée par un site de coupure PstI et par un site de coupure XhoI, caractérisé par la séquence d'aminoacides reproduite dans la figure 5, qui code pour la production de protéines qui conduisent à une activité aspartyl β-semialdéhyde deshydrogénase (asd) et à une dérégulation de l'aspartase-kinase (lys-C), contiennent dans le plasmide pCS2 dont la carte de suppressions doit être retirée de la figure 3, déposé dans Corynebactérium glutamicum sous le n^{o}DSM5086.

2. ADN recombinant dérivé du plasmide pCS2 selon la revendication 1 avec les cartes de suppression correspondant aux désignations pCS26 reproduites à la figure 3.

3. ADN recombinant dérivé du plasmide pCS2 selon la revendication 1 avec les cartes de suppression correspondant aux désignations pCS23 ou pCS223, reproduites dans la figure 3.

4. Procédé de production de la l-lysine par fermentation des microorganismes secrétant cet aminoacide, des genres Corynebactérium ou brevibactérium, caractérisé en ce que les micororganismes renferment le plasmide pCS2 conformément à la revendication 1 ou un ADN recombinant dérivé de celui-ci conformément aux revendications 2 et 3.

5. Procédé selon la revendication 4, caractérisé en ce que l'on met en oeuvre Corynebactérium glutamicum DSM 5086.

## Revendications (Revendications pour l'(les) Etat(s) contractant(s) suivant(s): ES)

1. Procédé d'obtention de L-lysine par fermentation de micro-organismes sécrétant cet aminoacide, des genres corynebactérium ou brevibactérium, et caractérisé en ce que les micro-organismes contiennent le plasmide pCS2 dont la carte de suppressions doit être retirée de la figure 3 et qui est déposé dans corynebactérium glutamicum sous le n^{o} DSM5086, contenant un fragment d'ADN formé essentiellement d'une séquence génétique ayant 2,1 kb de long, limitée par un site de coupure PstI et un site de coupure XhoI, caractérisé par la séquence d'amino-acides acides restituée dans la figure 5 qui code pour la production de protéine conduisant à une activité Asparty β semi-aldehyde deshydrogénase (asd) et à la dérégulation de l'aspartate-kinase (lys C).

2. Procédé selon la revendication 1, caractérisé en ce que les micro-organismes contiennent un ADN recombinant dérivé du plasmide pCS2 avec la carte de suppression correspondant à la dénomination pCS26, restituée dans la figure 3.

3. Procédé selon la revendication 1, caractérisé en ce que les micro-organismes contiennent un ADN recombinant dérivé du plasmide pCS2 avec les cartes de suppression correspondant aux désignations pCS23 ou pCS223, restituées dans la figure 3.

4. Procédé selon la revendication 1, caractérisé en ce que l'on met en oeuvre corynebactérium glutamicum DSM5086.
